Europäisches Patentamt

(19) European Patent Office

Office européen des brevets

(11) EP 0 649 837 B1

(12) FASCICULE DE BREVET EUROPEEN

(45) Date de publication et mention
de la délivrance du brevet:
**07.01.1998 Bulletin 1998/02**

(51) Int. Cl.⁶: $C07C\ 319/06$, $C07C\ 321/04$

(21) Numéro de dépôt: **94402265.6**

(22) Date de dépôt: **10.10.1994**

(54) **Synthèse du méthylmercaptan à partir du diméthyldisulfure**

Herstellung von Methylmercaptan aus Dimethyldisulfid

Synthesis of methyl mercaptan from dimethyldisulphide

(84) Etats contractants désignés:
**AT BE CH DE DK ES FR GB GR IE IT LI LU NL SE**

(30) Priorité: **20.10.1993 FR 9312491**

(43) Date de publication de la demande:
**26.04.1995 Bulletin 1995/17**

(73) Titulaire:
**ELF AQUITAINE PRODUCTION
92400 Courbevoie (FR)**

(72) Inventeurs:
 • **Cadot, Emmanuel
  F-75015 Paris (FR)**
 • **Lacroix, Michel
  F-96009 Lyon (FR)**
 • **Commarieu, Annie
  F-64000 Pau (FR)**
 • **Arretz, Emmanuel
  F-64000 Pau (FR)**

(74) Mandataire:
**Leboulenger, Jean et al
Elf Atochem S.A.,
Département Propriété Industrielle,
Cedex 42 - La Défense 10
92091 Paris la Défense (FR)**

(56) Documents cités:
DE-A- 1 903 968   FR-A- 1 386 654
FR-A- 2 008 331   US-A- 2 402 614

## Description

La présente invention concerne le domaine des mercaptans et a plus particulièrement pour objet un procédé de préparation sélective du méthylmercaptan à partir du diméthyldisulfure (DMDS) par réaction avec l'hydrogène sur un catalyseur constitué de sulfures de métaux de transition.

On sait que les sulfures des métaux de transition permettent de réaliser des réactions d'hydrogénation en présence de quantités importantes de molécules soufrées. En effet, contrairement aux catalyseurs d'hydrogénation classiques, comme les métaux, les phases sulfures ne sont pas empoisonnées par les molécules contenant du soufre. Leur utilisation pour la réduction catalytique de disulfures aromatiques en mercaptans est déjà décrite dans la littérature. Ainsi :

- la demande de brevet NL 6 402 424 revendique l'utilisation de sulfures métalliques, en particulier le sulfure de platine, pour la réduction du diphényldisulfure en phénylmercaptan ;
- pour cette même réaction, les brevets FR 2 008 331 et DE 1 903 968 préconisent les métaux Ni ou Co Raney, ainsi que les métaux Ru, Rh, Pt, Ir et Pd ou leurs sulfures ;
- selon la demande de brevet JP 56-81541, le sulfure de cobalt permet la réduction des composés aromatiques du types $RS_nR'$ en mercaptans RSH et R'SH, R et R' étant des radicaux phényle, p-nitrophényle ou 3,4-dichlorophényle.

Si ces procédés opèrent généralement à des températures de réaction relativement douces (env. 200°C), les pressions d'hydrogène sont par contre très élevées (50-100 bars) et des rapports catalyseur/réactif de l'ordre de 2 sont employés. De plus, la réaction est conduite en réacteur fermé et en milieu triphasique: gaz-liquide-solide.

La réduction catalytique sélective du diméthyldisulfure en méthylmercaptan n'apparaît pas avoir déjà été décrite. En revanche, l'hydrogénolyse totale du diméthyldisulfure est très utilisée pour la sulfuration des catalyseurs d'hydrotraitement qui est devenue l'application industrielle la plus importante du DMDS; dans ce cas, le DMDS est un précurseur d'hydrogène sulfuré qui est l'agent de sulfuration de ces catalyseurs. Les travaux relatifs à la sulfuration de catalyseurs d'hydrotraitement au moyen du DMDS mettent en évidence que, pendant la période de présulfuration de catalyseurs au Co-Mo et au Ni-Mo, la répartition des produits de décomposition du DMDS (méthylmercaptan, diméthylsulfure, hydrogène sulfuré, méthane) évolue en fonction de la température, une basse température (autour de 200°C) favorisant la formation du méthylmercaptan et celle du diméthyldisulfure. Par contre, à température plus élevée, l'hydrogénolyse du méthylmercaptan est rapide et conduit à la formation prépondérante d'hydrogène sulfuré et de méthane.

Le contrôle de l'hydrogénation du diméthyldisulfure pour produire uniquement du méthylmercaptan selon la réaction :

$$CH_3\text{-}SS\text{-}CH_3 + H_2 \rightarrow 2\ CH_3\text{-}SH$$

s'avère être difficile étant donné que ces deux composés possèdent des liaisons C-S sensibles à l'hydrogénolyse conduisant facilement à la formation d'hydrogène sulfuré et de méthane. De plus, la formation complémentaire de diméthylsulfure, observée sur des catalyseurs à base de métaux de transition (Co-Mo et Ni-Mo), est un facteur qui pénalise la production de méthylmercaptan.

Pour la sulfuration des catalyseurs d'hydrotraitement, on met en oeuvre des excès très importants d'hydrogène par rapport au diméthyldisulfure. En opérant avec de fortes proportions d'hydrogène, l'hydrogénation partielle du DMDS limitée au méthylmercaptan est défavorisée par rapport à une hydrogénolyse plus complète des composés organiques soufrés.

Cependant, en opérant avec des ratios hydrogène/DMDS beaucoup plus faibles, des réactions secondaires peuvent se produire sur le catalyseur et favoriser en particulier la formation de diméthylsulfure par la réaction :

$$2\ CH_3SH \rightarrow CH_3SCH_3 + H_2S$$

L'objectif recherché dans la présente invention consistait à trouver un procédé sélectif de fabrication du méthylmercaptan par hydrogénation catalytique du DMDS mettant en oeuvre des quantités d'hydrogène relativement faibles, de manière à faciliter l'exploitation industrielle d'un tel procédé pour éviter des recyclages importants de gaz et pour permettre une récupération efficace du méthylmercaptan, du fait de ses propriétés physiques particulières (point d'ébullition: +6°C à pression atmosphérique).

Les premiers travaux entrepris ont montré que les sulfures de métaux de transition ne réalisent pas de façon sélective l'hydrogénation du diméthyldisulfure en méthylmercaptan, même à des températures voisines ou inférieures à 200°C, à forte ou faible proportion d'hydrogène.

Les recherches poursuivies dans le but d'améliorer la sélectivité en méthylmercaptan ont conduit à mettre en évi-

2

dence que la présence d'eau, dans la réaction entre l'hydrogène et le DMDS, produit sur les propriétés catalytiques des catalyseurs un effet inattendu qui se traduit par un accroissement spectaculaire de la sélectivité en méthylmercaptan. Il a en outre été observé que la présence d'hydrogène sulfuré dans le milieu réactionnel apporte aussi un gain de sélectivité en méthylmercaptan ; l'effet de l'hydrogène sulfuré est cependant moins important que l'effet produit par l'eau.

La présente invention a donc pour objet un procédé de préparation sélective du méthylmercaptan par hydrogénation catalytique du diméthyldisulfure (DMDS) sur un catalyseur supporté sur un support minéral et à base de sulfure(s) d'au moins un métal de transition, caractérisé en ce qu'on opère en présence d'eau et/ou d'hydrogène sulfuré et en ce que le rapport molaire $H_2$/DMDS est compris entre 0,5 et 10.

Le catalyseur utilisé selon l'invention est constitué par un sulfure ou un mélange de sulfures d'au moins un métal de transition, ce ou ces sulfure(s) étant incorporé(s) à des supports minéraux. Les métaux de transition préférés sont le nickel, le cobalt, le molybdène et/ou le tungstène. Comme supports minéraux, peuvent être employés les solides poreux habituels tels que, par exemple, l'alumine, la silice, les silice-alumines, les zéolithes ou encore des charbons.

Dans les catalyseurs supportés, la teneur pondérale en métal de transition peut aller de 0,1 à 50 % et est, de préférence, comprise entre 0,5 et 30 %. Les catalyseurs supportés peuvent être préparés par imprégnation du support au moyen d'une solution aqueuse d'au moins un dérivé métallique tel que, par exemple, le nitrate de nickel ou de cobalt, le molybdate d'ammonium, le tungstate d'ammonium. Après imprégnation, les solides sont calcinés à 400-500°C sous air, sauf ceux à base de charbon qui sont calcinés sous azote. A l'issue de ce traitement, les différents métaux de transition se trouvent à l'état d'oxydes. Leur transformation en phases sulfures peut être réalisée de façon connue en soi, par exemple en soumettant les solides à l'action d'un mélange d'hydrogène et de diméthyldisulfure (rapport molaire $H_2$/DMDS compris entre 10 et 500) ou d'un mélange d'hydrogène et d'hydrogène sulfuré (rapport molaire $H_2/H_2S$ compris entre 1 et 500) à une température allant de 150 à 500°C. Les solides ainsi activés peuvent être utilisés soit sous forme d'extrudés, soit à l'état de poudre.

Pour une production continue de méthylmercaptan, le procédé selon l'invention est avantageusement mis en oeuvre dans un réacteur tubulaire dans lequel le catalyseur est disposé en lit fixe ou en lit mobile et dans lequel sont introduits les réactifs hydrogène et diméthyldisulfure, ainsi que l'eau ou l'hydrogène sulfuré, à des débits contrôlés.

Le procédé selon l'invention peut aussi être mis en oeuvre dans un réacteur agité. Ce mode de fonctionnement convient pour des productions discontinues de méthylmercaptan.

La réaction peut être conduite à pression atmosphérique ou à des pressions supérieures à la pression atmosphérique (jusqu'à environ 50 bars). La pression opératoire dépend, en règle générale, des caractéristiques du réacteur de synthèse et des éléments complémentaires définissant l'installation de production tels que l'alimentation en hydrogène, la technique de récupération du méthylmercaptan, le recyclage des effluents gazeux.

La réaction proprement dite peut avoir lieu dans un domaine assez large de température (50 à 400°C), mais dans la pratique il est préférable d'opérer à une température comprise entre 100 et 250°C de manière à obtenir le méthylmercaptan avec la sélectivité la plus élevée.

Le rapport molaire hydrogène/diméthyldisulfure à mettre en oeuvre est pour des raisons de contraintes industrielles, compris de préférence entre 0,8 et 4.

La proportion d'eau ou d'hydrogène sulfuré à intrbduire avec les réactifs hydrogène et diméthyldisulfure peut aller de 0,01 à 50 % en poids par rapport au diméthyldisulfure ; elle est de préférence comprise entre 0,1 % et 15 %.

Les exemples suivants illustrent l'invention sans la limiter.

## EXEMPLE 1

Les essais d'hydrogénolyse du DMDS rapportés dans cet exemple qui met en évidence l'effet de l'hydrogène sulfuré et celui de l'eau sur la formation sélective du méthylmercaptan, ont été réalisés dans des conditions de faibles pressions partielles des réactifs dilués dans l'azote.

### a) *Catalyseurs*

Les catalyseurs d'origine commerciale avaient été préparés à partir d'une même alumine activée (surface spécifique : 256 $m^2.g^{-1}$ ; volume poreux : 0,6 $ml.g^{-1}$ ; teneur en impuretés : 1700 ppm). Ils se présentaient sous forme d'extrudés et les constituants métalliques étaient à l'état d'oxydes. La nature des constituants métalliques et leur proportions sont données dans le tableau suivant.

TABLEAU 1

| CONSTITUANT METALLIQUE | TENEUR PONDERALE (%) | CONCENTRATION ($10^{-4}$ mol/g cata.) |
|---|---|---|
| Ni | 2,4 | 4,0 |
| Co | 2,4 | 4,0 |
| Mo | 9,3 | 9,7 |
| NiMo | Ni : 2,4 | 4,0 |
| | Mo : 9,3 | 9,7 |
| NiW | Ni : 2,4 | 4,0 |
| | W : 16,6 | 9,0 |
| CoMo | Co : 2,4 | 4,0 |
| | Mo : 9,3 | 9,7 |

La sulfuration des catalyseurs a été effectuée par traitement à 400°C pendant 4 heures au moyen d'un mélange hydrogène sulfuré-hydrogène (contenant 15 % molaire d'$H_2S$) au débit horaire de 2 litres de mélange par gramme de catalyseur.

Pour simplifier l'exposé des essais d'hydrogénation suivants, les catalyseurs sulfurés seront maintenant identifiés dans les tableaux par la seule indication du ou des métaux de transition, étant précisé que les masses de catalyseur indiquées s'entendent du total : sulfure(s) métallique(s) + support $Al_2O_3$.

## b) *Hydrogénation du diméthyldisulfure*

Les essais ont été effectués à pression atmosphérique dans un réacteur tubulaire en verre contenant dans sa partie centrale le catalyseur broyé. Après avoir été purgé à l'azote, le réacteur logé dans un four électrique était porté à la température réactionnelle. Les réactifs (hydrogène et DMDS) dilués dans l'azote étaient ensuite introduits dans le réacteur avec un débit gazeux total de 60 ml par minute. La pression partielle de DMDS était fixée à 2 kPa et celle d'hydrogène à 4 kPa, c'est-à-dire que le ratio molaire $H_2$/DMDS était égal à 2. La température de réaction était de 200°C.

Les effluents gazeux sortant du réacteur étaient directement analysés par chromatographie en phase gazeuse au moyen d'un chromatographe dont la vanne d'injection était alimentée en continu par l'effluent. Les catalyseurs du tableau 1 ainsi que l'alumine ayant servi de support à ces différents catalyseurs ont été testés dans les mêmes conditions. Les résultats reportés dans le Tableau 2 donnent les sélectivités relatives du méthylmercaptan (MeSH) et du diméthylsulfure (DMS) pour des essais qui ont été effectués à des conversions de DMDS se situant entre 35 et 45 %.

TABLEAU 2

| CATALYSEUR | | CONVERSION (%) | SELECTIVITES RELATIVES (%) | |
|---|---|---|---|---|
| Métal | Masse (g) | DMDS | MeSH | DMS |
| néant | 0,117 | 44 | 21 | 79 |
| Mo | 0,077 | 37 | 19,6 | 80,4 |
| Ni | 0,061 | 36 | 11,5 | 88,5 |
| Co | 0,050 | 41 | 13,4 | 86,6 |
| NiMo | 0,118 | 45 | 55,3 | 44,7 |
| CoMo | 0,076 | 38 | 31,1 | 68,9 |
| NiW | 0,119 | 35 | 55,5 | 44,5 |

## c) *Hydrogénation du diméthyldisulfure en présence d'hydrogène sulfuré*

La seule modification au protocole opératoire de l'exemple 1b était l'addition de 0,1 mole d'hydrogène sulfuré par mole de DMDS. Le tableau 3 suivant rassemble les résultats obtenus dans ces conditions et à une température de 200°C.

TABLEAU 3

| CATALYSEUR | | CONVERSION (%) | SELECTIVITES RELATIVES (%) | |
|---|---|---|---|---|
| Métal | Masse (g) | DMDS | MeSH | DMS |
| néant | 0,120 | 51 | 73 | 27 |
| Mo | 0,080 | 19 | 100 | 0 |
| Ni | 0,060 | 14 | 85 | 15 |
| NiMo | 0,105 | 32 | 99 | 1 |

## d) *Hydrogénation du diméthyldisulfure en présence d'eau*

La seule modification apportée au protocole opératoire de l'exemple 1b était l'addition de 0,1 mole d'eau par mole de DMDS. Les résultats qui ont été obtenus dans ces conditions et à une température de 200°C sont présentés dans le tableau 4.

TABLEAU 4

| CATALYSEUR | | CONVERSION (%) | SELECTIVITES (%) | |
|---|---|---|---|---|
| Métaux | Masse (g) | DMDS | MeSH | DMS |
| NiMo | 0,118 | 41 | 100 | 0 |
| CoMo | 0,076 | 11 | 100 | 0 |
| NiW | 0,119 | 27 | 100 | 0 |

Des essais complémentaires en présence d'eau ont été réalisés avec le catalyseur ayant donné les meilleurs résultats (Ni-Mo/Alumine).

En travaillant à différents taux de conversion du DMDS par variation de la masse de catalyseur, on a obtenu les résultats résumés dans le tableau 5. Même à forte conversion (91 %), la sélectivité en méthylmercaptan se maintenait à un niveau élevé (95,6 %).

TABLEAU 5

| Conversion du DMDS (%) | 29 | 40 | 58 | 79,2 | 91 |
|---|---|---|---|---|---|
| Sélectivité MeSH (%) | 100 | 100 | 98 | 97 | 95,6 |

En faisant varier la proportion d'eau ajoutée, on a obtenu les résultats résumés dans le tableau 6. On observe que ni la conversion, ni la sélectivité n'étaient modifiées par une augmentation de la proportion d'eau.

TABLEAU 6

| Rapport molaire $H_2O$/DMDS | 0,13 | 0,27 | 0,40 | 0,67 | 1,20 |
|---|---|---|---|---|---|
| Conversion du DMDS (%) | 42 | 42 | 41 | 39 | 39 |
| Sélectivité MeSH (%) | 100 | 100 | 100 | 100 | 100 |

**EXEMPLE 2**

Les essais d'hydrogénolyse du DMDS en présence d'eau rapportés dans cet exemple ont été réalisés à partir du même catalyseur commercial Ni-Mo/alumine qu'à l'exemple 1 (catalyseur HR 346 de PROCATALYSE).

La sulfuration du catalyseur Ni-Mo/Alumine (50 g) a été faite directement dans le réacteur servant à effectuer l'hydrogénolyse du DMDS, par traitement à 400°C pendant 4 heures au moyen d'un mélange d'hydrogène sulfuré-hydrogène (contenant 15 % molaire d'$H_2S$) au débit horaire de 2 litres de mélange par gramme de catalyseur.

Les essais d'hydrogénolyse ont été réalisés à pression atmosphérique dans un réacteur tubulaire en acier Inox (diamètre interne: 25 mm) muni d'une gaine thermométrique et logé au centre d'un four électrique à trois zones de chauffe indépendantes. Le DMDS et l'eau injectés à débits contrôlés sous forme liquide et l'hydrogène gazeux étaient introduits à la partie supérieure du réacteur qui servait de zone de préchauffage.

Les effluents de réaction étaient maintenus gazeux par chauffage des lignes de circulation à l'extérieur du réacteur et étaient envoyés dans une vanne d'injection couplée à un chromatographe qui analysait directement ces effluents.

Le tableau 7 suivant rassemble les résultats obtenus en faisant varier la température (150, 175 et 200°C) et le débit horaire de DMDS (42 à 166 g/h), mais en maintenant constants les ratios molaires $H_2$/DMDS et DMDS/$H_2O$ à 2,1 et 2,7 respectivement.

TABLEAU 7

| TEMPERATURE (°C) | DMDS g/h | CONVERSION DU DMDS (%) | SELECTIVITE EN MeSH (%) |
|---|---|---|---|
| 150 | 63 | 71,8 | > 99,5 |
| 150 | 125 | 60 | > 99,5 |
| 175 | 63 | 100 | > 99,5 |
| 175 | 83 | 92,2 | > 99,5 |
| 175 | 104 | 91 | > 99,5 |
| 175 | 166 | 87,6 | > 99,5 |
| 200 | 42 | 100 | ≥ 99,5 |
| 200 | 63 | 100 | ≥ 99,5 |
| 200 | 104 | 98,4 | ≥ 99,5 |
| 200 | 125 | 93,2 | ≥ 99,5 |

Le tableau 8 suivant rassemble les résultats obtenus à 175°C en faisant varier la proportion d'hydrogène pour un même débit du DMDS (166 g/h) et un rapport molaire DMDS/$H_2O$ maintenu constant à 2,7.

TABLEAU 8

| RAPPORT MOLAIRE $H_2$/DMDS | CONVERSION DU DMDS (%) | SELECTIVITE EN MeSH (%) |
|---|---|---|
| 1,2 | 65,3 | >99,5 |
| 1,4 | 72,5 | >99,5 |
| 1,7 | 80,0 | >99,5 |
| 2,1 | 87,6 | >99,5 |

**Revendications**

1. Procédé de préparation sélective du méthylmercaptan par hydrogénation catalytique du diméthyldisulfure (DMDS) sur un catalyseur supporté sur un support minéral et à base de sulfure(s) d'au moins un métal de transition, caractérisé en ce qu'on opère en présence d'eau et/ou d'hydrogène sulfuré et en ce que le rapport molaire $H_2$/DMDS est compris entre 0,5 et 10.

**2.** Procédé selon la revendication 1, dans lequel la proportion d'eau ou d'hydrogène sulfuré va de 0,01 à 50 % en poids par rapport au diméthyldisulfure et est de préférence comprise entre 0,1 et 15 %.

**3.** Procédé selon la revendication 1 ou 2, caractérisé en ce que le rapport molaire $H_2$/DMDS est compris entre 0,8 et 4.

**4.** Procédé selon l'une des revendications 1 à 3, dans lequel on opère à une température allant de 50 à 400°C, de préférence comprise entre 100 et 250°C.

**5.** Procédé selon l'une des revendications 1 à 4, dans lequel on opère à la pression atmosphérique ou à une pression supérieure pouvant aller jusqu'à 50 bars.

**6.** Procédé selon l'une des revendications 1 à 5, dans lequel le catalyseur est à base de sulfure(s) de nickel, cobalt, molybdène et/ou tungstène.

**7.** Procédé selon l'une des revendications 1 à 5, dans lequel le catalyseur est constitué de sulfures de nickel et de molybdène sur un support d'alumine.

## Claims

**1.** Process for the selective preparation of methyl mercaptan by catalytic hydrogenation of dimethyl disulphide (DMDS) on a catalyst supported on an inorganic support and based on sulphide(s) of at least one transition metal, characterized in that the process is performed in the presence of water and/or hydrogen sulphide, and in that the $H_2$/DMDS molar ratio is between 0.5 and 10.

**2.** Process according to Claim 1, in which the proportion of water or of hydrogen sulphide ranges from 0.01 to 50 % by weight relative to the dimethyl disulphide, and is preferably between 0.1 and 15 %.

**3.** Process according to Claim 1 or 2, characterized in that the $H_2$/DMDS molar ratio is between 0.8 and 4.

**4.** Process according to one of Claims 1 to 3, in which the process is performed at a temperature ranging from 50 to 400°C, preferably between 100 and 250°C.

**5.** Process according to one of Claims 1 to 4, in which the process is performed at atmospheric pressure or at a greater pressure which may range up to 50 bar.

**6.** Process according to one of Claims 1 to 5, in which the catalyst is based on sulphide(s) of nickel, cobalt, molybdenum and/or tungsten.

**7.** Process according to one of Claims 1 to 5, in which the catalyst consists of nickel and molybdenum sulphides on an alumina support.

## Patentansprüche

**1.** Verfahren zur selektiven Herstellung von Methylmercaptan durch katalytische Hydrierung von Dimethyldisulfid (DMDS) an einem Trägerkatalysator auf anorganischem Trägermaterial auf Basis von Sulfid(en) mindestens eines Übergangsmetalles, dadurch gekennzeichnet, daß man in Gegenwart von Wasser und/oder Schwefelwasserstoff verfährt und das $H_2$/DMDS-Molverhältnis zwischen 0,5 und 10 liegt.

**2.** Verfahren nach Anspruch 1, dadurch gekennzeichnet, daß die Wasser- oder Schwefelwasserstoffmenge zwischen 0,01 und 50 Gew.-%, vorzugsweise zwischen 0,1 und 15 Gew.-% liegt, bezogen auf das Dimethyldisulfid.

**3.** Verfahren nach Anspruch 1 oder 2, dadurch gekennzeichnet, daß das $H_2$/DMDS-Molverhältnis zwischen 0,8 und 4 liegt.

**4.** Verfahren nach einem der Ansprüche 1 bis 3, bei dem man bei einer Temperatur zwischen 50 und 400 °C, vorzugsweise zwischen 100 und 250 °C, verfährt.

5. Verfahren nach einem der Ansprüche 1 bis 4, bei dem man bei Atmosphärendruck oder bei einem erhöhten Druck von bis zu 50 bar arbeitet.

6. Verfahren nach einem der Ansprüche 1 bis 5, bei dem der Katalysator auf Basis von Nickel-, Cobalt-, Molybdän- und/oder Wolframsulfid vorliegt.

7. Verfahren nach einem der Ansprüche 1 bis 5, bei dem der Katalysator aus Nickel- und Molybdänsulfid auf einem Aluminiumoxidträger besteht.